# EUROPEAN PATENT APPLICATION

(11) **EP 1 955 656 A1**
(43) Date of publication of application: **13.08.2008**
(21) Application number: 08002515.8
(22) Date of filing: 12.02.2008
(51) Int. Cl.: A61B 10/00, G01N 33/483

(54) **Constructive disposition applied to sample collector for the purposes of parasitological feces examination**

(30) Priority: 12.02.2007 BR MU8700248
(71) Applicant: Lapenna, José Carlos, 13313-700 Itú (BR)
(72) Inventor: Lapenna, José Carlos, 13313-700 Itú (BR)
(74) Representative: Thierry-Carstensen, Ole Jean

(57) **Abstract**

The present application concerns a new construction arrangement applied to a sample collector for the purposes of parasitological feces examinations, more precisely to one new construction format that is manufactured with a reduced number of parts, helping to reduce the cost, since this is a product that is disposed after use; a collector (1) including a container (2) that has a cover (3) with a central conical sector (4) from the top of which a cylindrical duct (5) is developed that may be closed with a cap (6); provided with a concave filter element (8), formed by a polyester screen or fabric with about 220 to 280µ mesh, preferably 266µ, presenting the same inner curve radius of the conical part (4) of the cover, attached to the same by means of a fitting (E) or other equivalent means.

## Description

The present application refers to the use of a new constructive disposition, as a device applied to sample collector for the purposes of parasitological feces examination, more precisely to one new construction format which aims to ease the work of the laboratory technician work during the collection, transportation, dilution and filtering operation, in order to obtain the sample by agitation of the container, and at the end, pouring some drops on the slide or blade for the microscopic examination; said new collector is manufactured with a reduced number of parts, which results in low costs, since it is a product to be disposed after use.

In general, the parasitological feces examinations, performed in clinic analysis laboratories, are based on a procedure which includes the process from the collection of the material up to the filtering of the same in order to obtain a sample to be forwarded to the analysis equipment.

Currently, it is known that the sample is obtained with conical filter elements, consisting of high density polyethylene screen or fabric with about 500µ mesh. Even though this is a dimensionally acceptable mesh, it is not considered ideal for obtaining the sample, and it is not reliable for the analysis process.

This occurs because the polyethylene mesh presents a variation as regards correct weft spacing, such as imperfections that occur during the mesh injecting process, resulting in whole areas without perforation, therefore affecting the performance of the filter element.

Another factor of high importance concerns the bio-safety concept related to the clinical analysis field, since the handled material is very infecting, and may present risks to the professional handling of the same. The present applicant has already filed an application at the responsible agency - INPI - under number MU 8402055-5, presenting an improved collector, with a construction design and concept that is different from those in the conventional market and designed as a jar shaped container, with a filter element assembled inside the same, with a side part formed by a polyester frame with about 150 to 300µ mesh, having in the lower part a stem shaped element to which end is applied the collector, where the filtered material precipitates and settles in the grooves or holes, remaining also submerged in conservation liquid.

Such construction has been successful as regards the procedure in clinical analysis laboratories. Because it synthesises the parasitological feces examination, from its collection through the conservation, dilution, filtering, and providing very clean sediment for microscopic analysis, it performs more examinations in a reduced physical space and time, at an extremely low cost.

Therefore, the applicant, operating in the area and concerned with improving parasitological examination quality, conceived and designed a new collector model that improves the above mentioned model, since it effectively enhances not only the laboratory technician work, but also the obtained result.

The improved collector, object of the present invention, consists of a reduced number of parts, which represent lower costs in relation to those currently available on the market. In this sense, the collector includes a receiving container with capacity around 35 ml, a cover consisting of a central conical sector covered by a cylindrical duct where the cap is attached, and a lower cylindrical sector with internal thread, which may be attached to the receiving container opening. A concave filter element is designed with polyester screen or fabric with about 220 to 280µ mesh, preferably 266µ, which is attached to the internal surface of the conical part of the cover by means of fitting or other proper means.

Such construction, compared to the previously described one, maintains the excellent filtering of the material to be analysed, and also provides a reduced cost solution, since such collection devices are disposed after use.

In order to complement the present description for better understanding the features of the invention and in accordance with its preferred practical embodiment, the description is accompanied by a set of drawings, as an example, although not limited to, of the following:

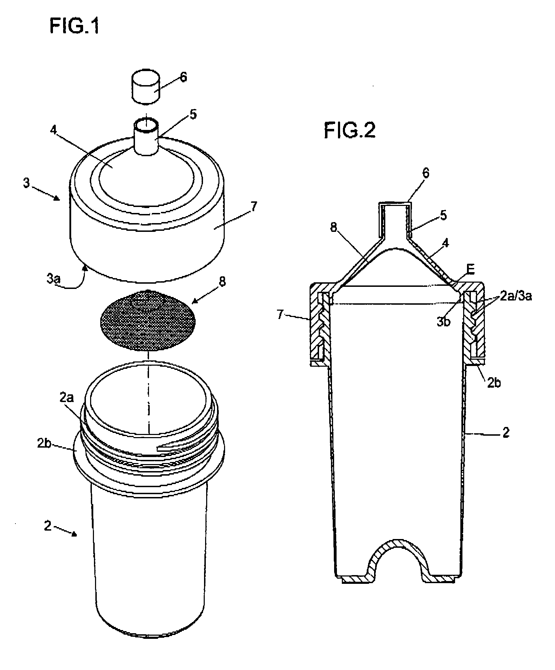

Figure 1 represents an exploded view of the collector, according to its preferred embodiment, showing the assembly components;
Fig. 2 shows one section of the assembled collector, as presented in figure 1,
Fig. 3 to 6 show the sequence for using the collector, and
Fig. 7 and 8 represent a construction variation of the container with a blade, designed for a certain type of material.

As regards the presented drawings, the present application refers to a new "CONSTRUCTIVE DISPOSITION APPLIED TO SAMPLE COLLECTOR FOR THE PURPOSES OF PARASITOLOGICAL FECES EXAMINATION", more precisely one collector (1) which includes a container (2), with capacity around 35 ml, preferably in cylindrical shape, whose top external part is provided with thread (2a) and flange (2b); said thread (2a) receives one cover (3) with thread (3a) and has, as sealing element between parts (2) and (3), a circular ring (3b).

According to the present invention, the cover (3) has a central conical sector (4) from which top is developed a cylindrical duct (5) that may be closed with a cap (6); a larger conical sector base (4), the cover of which develops a cylindrical sector (7), with the inner thread (3a).

The collector (1) has a concave filter element (8), formed by polyester screen or fabric with about 220 to 288µ mesh, preferably 266µ, this element semi-following the inner curve of the conical part (4) at the cover, attached to the same by fitting into the groove (E) especially designed to receive and accommodate the element (8) safely in order to maintain the mesh edge attached to the inner face of the cover.

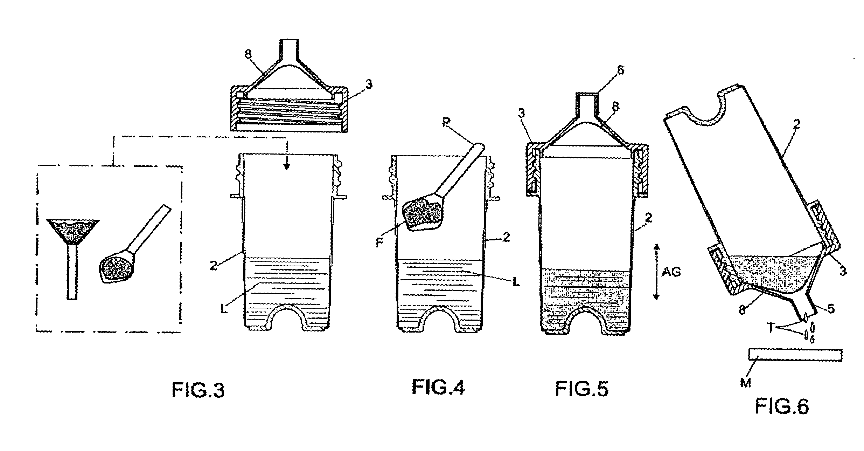

The collector utilisation format, represented in Fig. 3 to 6, shows that the container (2), already supplied with conservation liquid (L), is separated from the cover in order to receive a part of the feces (F), collected with a blade (P), of any shape.

Subsequently, the container (2) is closed with the cover, still closed by the cap (6); whereupon, the collector is agitated (AG) until the feces are diluted into the liquid (L).

After this, the cap is removed and the collector is turned over to allow some drops (T) of the obtained dilution passing by the filter element (8), and forwarded, in blades (M), to the proper clinic analysis.

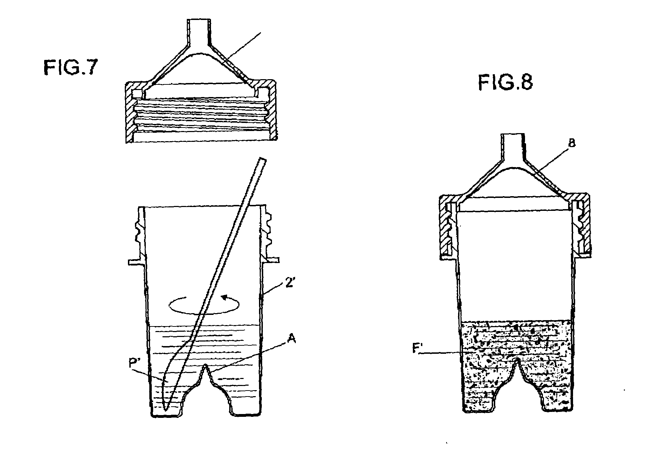

In a construction variation, Fig. 7 and 8, more particularly applied to the case of hard feces, which usually produces "poor" samples due to the lack of dilution, the container bottom (2') includes a funneled extension (A), which will be used as scrapping means and support for removing the feces sample (P) from the blade (P'), this shape also allowing macerating the feces sample (F') until complete dilution, thus solving the problem concerning "poor" samples due to lack of dilution.

Even though the above describes the preferred embodiment of the present invention, any modifications and/or changes shall be understood as included and in the scope of the invention, perfectly included in the criteria defining the invention, i.e., the combination and modification of the already known elements in new shape or arrangement, resulting in functional improvement for its use or manufacture.

## Claims

1. Constructive disposition applied to sample collector for the purposes of parasitological feces examination, more particularly a collector (1) which includes a container (2), with capacity around 35 ml, preferably in cylindrical shape, the top external part of which is provided with thread (2a) and flange (2b); said thread (2a) receives a cover (3) with the thread (3a) and has, as sealing element between the parts (2) and (3), a circular ring (3b); the cover includes a central conical sector (4) from which top is developed a cylindrical duct (5) that may be closed with a cap (6); from the larger base of the conical sector (4), said cover develops a cylindrical sector (7), with the inner thread (3a); collector is provided with blades (P), with any shape, which take the feces samples (F) to the collector (1); said collector **characterised in that** one concave filter element (8), formed by a polyester screen or fabric with about 220 to 280µ mesh, preferably 266µ, presenting a semi-following of the inner curve of the conical part (4) of the cover, attached to it by means of a fitting (E) or other equivalent means.

2. Constructive disposition applied to sample collector for the purposes of parasitological feces examination" according to claim 1 and in an alternate construction variation, particularly applied to the case of hard feces or with little dilution (F'), **characterised in that** the container bottom includes a funneled extension (A) used as scrapping means and support for removing the feces sample (F') from the blade (P').
